(19) Europäisches Patentamt
European Patent Office
Office européen des brevets

(11) EP 1 861 355 B1

(12) EUROPÄISCHE PATENTSCHRIFT

(45) Veröffentlichungstag und Bekanntmachung des Hinweises auf die Patenterteilung:
**17.09.2008 Patentblatt 2008/38**

(21) Anmeldenummer: **06708754.4**

(22) Anmeldetag: **14.03.2006**

(51) Int Cl.:
*C07C 209/16* (2006.01) *C07C 211/05* (2006.01)

(86) Internationale Anmeldenummer:
**PCT/EP2006/060701**

(87) Internationale Veröffentlichungsnummer:
**WO 2006/097468 (21.09.2006 Gazette 2006/38)**

# (54) VERFAHREN ZUR HERSTELLUNG EINES ETHYLAMINS

METHOD FOR PRODUCING AN ETHYLAMINE

PROCEDE DE FABRICATION D'UNE ETHYLAMINE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **15.03.2005 DE 102005012209**

(43) Veröffentlichungstag der Anmeldung:
**05.12.2007 Patentblatt 2007/49**

(73) Patentinhaber: **BASF SE**
**67056 Ludwigshafen (DE)**

(72) Erfinder:
- **GERLACH, Till**
  **67071 Ludwigshafen (DE)**
- **HAESE, Frank**
  **63128 Dietzenbach (DE)**
- **MEIER, Anton**
  **67134 Birkenheide (DE)**
- **MELDER, Johann-Peter**
  **67459 Böhl-Iggelheim (DE)**
- **RÜTTER, Heinz**
  **B-2950 Kapellen (BE)**

(56) Entgegenhaltungen:
**WO-A-20/05063681**
**US-A- 4 149 998**
**US-A- 4 760 190**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 861 355 B1

## Beschreibung

Die vorliegende Erfindung betrifft ein Verfahren zur Herstellung eines Ethylamins durch Umsetzung von Ethanol mit Ammoniak, einem primären Amin oder einem sekundären Amin in Gegenwart von Wasserstoff und eines Heterogenkatalysators.

Verfahren zur Herstellung eines Ethylamins durch Umsetzung von Ethanol mit Ammoniak, einem primären Amin oder einem sekundären Amin sind aus der Literatur bekannt, siehe z.B. Ullmann's Encyclopedia of Industrial Chemistry, 6th Edition, 2000 electronic release, ‚aliphatic Amines: Production from alcohols'.

Das eingesetzte Ethanol kann synthetisch hergestellt werden, etwa durch Hydratisierung von Ethylen. Als Alternative zu synthetischem Ethanol bietet sich biologisch oder biochemisch, insbesondere fermentativ hergestelltes, sogenanntes Bio-Ethanol, an. Dieses wird aus regenerativen Quellen hergestellt und ist damit aus ökologischen Gründen vorteilhaft. Zudem weist Bio-Ethanol teilweise einen günstigeren Preis auf als synthetisches Ethanol.

Beim Einsatz von Bio-Ethanol an vielen Aminierungs-Katalysatoren wurde jedoch eine deutlich schnellere Katalysatordeaktivierung beobachtet als dies bei Einsatz von synthetischem Ethanol bekannt war.

Aufgrund der schnelleren Deaktivierung muss die Synthese häufiger unterbrochen werden, um den Katalysator zu wechseln. Dies führt zu Produktionsausfall, erhöhten Kosten für Katalysator und Katalysatorwechsel und einem erhöhten Personalaufwand verbunden mit erhöhtem Unfallrisiko.

Wird Bio-Ethanol in Aminierungsverfahren eingesetzt, belegt sich, wie erfindungsgemäß erkannt wurde, die katalytisch aktive Metalloberfläche des jeweiligen Heterogenkatalysators mit der Zeit mehr und mehr mit dem/den durch den Bio-Alkohol eingetragenen Schwefel bzw. Schwefelverbindungen. Dies führt zu einer beschleunigten Katalysatordeaktivierung und damit zu einer deutlichen Beeinträchtigung der Wirtschaftlichkeit des jeweiligen Prozesses.

Synthetisches Ethanol weist im allgemeinen einen Gehalt an Schwefel und/oder schwefelhaltigen Verbindungen von ≤ 0,1 Gew.-ppm (berechnet S), z.B. bestimmt nach Wickbold (DIN EN 41), auf.

Die WO-Patentanmeldung mit dem Aktenzeichen PCT/EP/04/014591 vom 22.12.04 (BASF AG) betrifft ein Verfahren zur Abreicherung von Schwefel und/oder einer schwefelhaltigen Verbindung aus einer biochemisch hergestellten organischen Verbindung, wobei man die entsprechende organische Verbindung mit einem Adsorber in Kontakt bringt.

Die WO-Patentanmeldung mit dem Aktenzeichen PCT/EP/04/014588 vom 22.12.04 (BASF AG) betrifft ein Verfahren zur Herstellung eines Ethylamins durch Umsetzung von Ethanol mit Ammoniak, einem primären Amin oder einem sekundären Amin in Gegenwart von Wasserstoff und eines Heterogenkatalysators, wobei man ein biochemisch hergestelltes Ethanol (Bio-Ethanol) einsetzt, in welchem zuvor durch In-Kontakt-Bringen mit einem Adsorber Schwefel und/oder schwefelhaltige Verbindungen abgereichert wurden.

Die deutsche Patentanmeldung Nr. 102005010328.6 vom 03.03.05 (BASF AG) betrifft ein Verfahren zur Abreicherung von Schwefel und/oder einer schwefelhaltigen Verbindung aus einem Zucker, einem Zuckeralkohol und/oder einer Zuckersäure, wobei man den Zucker, Zuckeralkohol und/oder die Zuckersäure in flüssiger Phase mit einem Adsorber in Kontakt bringt.

US 4,760,190 betrifft die Herstellung eines Ethylamins aus Ethanol und Ammoniak in Gegenwart spezifischer dotierter Co- und/oder Ni-Alumina-Katalysatoren.

Der vorliegenden Erfindung lag die Aufgabe zugrunde, ein verbessertes wirtschaftliches Verfahren zur Herstellung eines Ethylamins ausgehend von Bio-Ethanol aufzufinden, durch das in hoher Ausbeute, Raum-Zeit-Ausbeute und Selektivität entsprechende Ethylamine, insbesondere Mono-, Di- und/oder Triethylamin, erhalten werden. Insbesondere sollte das Verfahren verlängerte Katalysatorstandzeiten bei der Synthese der Ethylamine ermöglichen.

**(Raum-Zeit-Ausbeuten werden angegeben in ‚Produktmenge / (Katalysatorvolumen • Zeit)' ($kg/(l_{Kat.}$ • h)) und/oder ‚Produktmenge / (Reaktorvolumen • Zeit)' ($kg/(l_{Reaktor}$ • h)).**

Demgemäß wurde ein Verfahren zur Herstellung eines Ethylamins durch Umsetzung von Ethanol mit Ammoniak, einem primären Amin und/oder einem sekundären Amin in Gegenwart von Wasserstoff und eines Heterogen-Hydrier-/Dehydrier-Katalysators gefunden, welches dadurch gekennzeichnet ist, dass man ein biochemisch oder biologisch hergestelltes Ethanol (= Bio-Ethanol) einsetzt, der Katalysator in seiner katalytisch aktiven Masse vor der Aktivierung mit Wasserstoff 20 bis 65 Gew.-% Zirkoniumdioxid ($ZrO_2$) und/oder Aluminiumoxid ($Al_2O_3$) als Trägermaterial, 1 bis 30 Gew.-% sauerstoffhaltige Verbindungen des Kupfers, berechnet als CuO, und 21 bis 70 Gew.-% sauerstoffhaltige Verbindungen des Nickels, berechnet als NiO, enthält und nach Aktivierung mit Wasserstoff eine CO-Aufnahmekapazität von > 110 μmol CO / g Katalysator aufweist, wobei die Aktivierung des Katalysators mit Wasserstoff bei einer Temperatur

im Bereich von 100 bis 500°C über einen Zeitraum von mindestens 25 Min. erfolgt.

Das erfindungsgemäß eingesetzte Bio-Ethanol wird im Allgemeinen aus Agrarprodukten wie Melasse, Rohrzuckersaft, Maisstärke oder aus Produkten der Holzverzuckerung und aus Sulfitablaugen durch Fermentation erzeugt.

Bevorzugt wird Bio-Ethanol eingesetzt, dass durch Fermentation von Glukose unter $CO_2$-Abspaltung erhalten wurde (K. Weissermel und H.-J. Arpe, Industrial Organic Chemistry, Wiley-VCH, Weinheim, 2003, p. 194; Electronic Version of Sixth Edition of Ullmann's Encyclopedia of Industrial Chemistry, 2000, Chapter Ethanol, Paragraph Fermentation).

Das Ethanol wird in der Regel durch Destillationsverfahren aus den Fermentationsbrühen gewonnen: Electronic Version of Sixth Edition of Ullmann's Encyclopedia of Industrial Chemistry, 2000, Chapter Ethanol, Paragraph ‚Recovery and Purification'.

Insbesondere und vorteilhaft wird im erfindungsgemäßen Verfahren ein biologisch oder biochemisch hergestelltes Ethanol (Bio-Ethanol) einsetzt, in welchem nicht zuvor, z.B. durch In-Kontakt-Bringen mit einem Adsorber, wie z.B. Kieselgel, ein aktiviertes Aluminiumoxid, ein Zeolith mit hydrophilen Eigenschaften, eine Aktivkohle oder ein Kohlenstoffmolsieb, Schwefel und/oder schwefelhaltige Verbindungen abgereichert wurden.

Im erfindungsgemäßen Verfahren wird bevorzugt ein Bio-Ethanol eingesetzt, das einen Gehalt an Schwefel und/oder schwefelhaltigen Verbindungen von $\geq$ 0,5 Gew.-ppm, oder $\geq$ 1 Gew.-ppm, oder $\geq$ 2 Gew.-ppm, oder $\geq$ 5 Gew.-ppm, oder $\geq$ 10 Gew.-ppm, (jeweils berechnet S), z.B. bestimmt nach Wickbold (DIN EN 41) (bei S-Gehalten $\leq$ 2 Gew.-ppm) bzw. coulometrisch bestimmt nach DIN 51400 Teil 7 (bei S-Gehalten > 2 Gew.-ppm), aufweist.
Der Gehalt an Schwefel und/oder schwefelhaltigen Verbindungen kann z.B. bis 10 Gew.-ppm, bis 50 Gew.-ppm, bis 100 Gew.-ppm oder bis 200 Gew.-ppm, (jeweils berechnet S), z.B. coulometrisch bestimmt nach DIN 51400 Teil 7, betragen.

Besonders bevorzugt wird ein Bio-Ethanol eingesetzt, das einen Gehalt an Schwefel und/oder schwefelhaltigen Verbindungen im Bereich von $\geq$ 0,5 bis 2 Gew.-ppm (berechnet S), z.B. bestimmt nach Wickbold (DIN EN 41), aufweist.

Bei den schwefelhaltigen Verbindungen handelt es sich um anorganische Verbindungen, wie Sulfate, Sulfite, und/oder organische Verbindungen, insbesondere um symmetrische und/oder unsymmetrische $C_{2-10}$-Dialkylsulfide, besonders $C_{2-6}$-Dialkylsulfide, wie Diethylsulfid, Di-n-propylsulfid, Di-isopropylsulfid, ganz besonders Dimethylsulfid, $C_{2-10}$-Dialkylsulfoxide, wie Dimethylsulfoxid, Diethylsulfoxid, Dipropylsulfoxid, 3-Methylthio-1-propanol und/oder S-haltigen Aminosäuren, wie Methionin und S-Methylmethionin.

In besonderen Ausführungen wird ein Bio-Ethanol eingesetzt, das zusätzlich zum o.g. Gehalt an Schwefel und/oder schwefelhaltigen Verbindungen
einen Gehalt an $C_{3-4}$-Alkanolen im Bereich von 1 - 5000 Gew.-ppm, besonders 5 - 3000 Gew.-ppm, ganz besonders 10 - 2000 Gew.-ppm,
einen Gehalt an Methanol im Bereich von 1 - 5000 Gew.-ppm, besonders 5-3000 Gew.-ppm, ganz besonders 20 - 1000 Gew.-ppm,
einen Gehalt an Ethylacetat im Bereich von 1 - 5000 Gew.-ppm, besonders 5 - 3000 Gew.-ppm, ganz besonders 10 - 2000 Gew.-ppm, und
einen Gehalt an 3-Methyl-butanol-1 im Bereich von 1 - 5000 Gew.-ppm, besonders 5 - 3000 Gew.-ppm, ganz besonders 10- 2000 Gew.-ppm
aufweist.

Der Gehalt an $C_{3-4}$-Alkanolen (wie n-Propanol, iso-Propanol, n-Butanol, iso-Butanol, tert.-Butanol), Methanol, Ethylacetat und 3-Methyl-butanol-1 wird z.B. bestimmt mittels Gaschromatographie (30 m DB-WAX-Säule, Innendurchmesser: 0,32 mm, Filmdicke: 0,25 $\mu$m, FID-Detektor, Temperaturprogramm: 35°C (5 Min.), 10°C/Min. Aufheizrate, 200°C (8 Min.)).

Der im erfindungsgemäßen Verfahren eingesetzte Katalysator enthält Cu und Ni. Die Katalysatoren können dotiert sein, etwa mit Ag, Zn, In, Mn, Alkalimetallen (Li, Na, Ka, Rb, Cs) und/oder Mo.

Als Trägermaterial für diese Aktivmetalle werden Aluminiumoxid (gamma, delta, theta, alpha, kappa, chi oder Mischungen daraus), Zirkoniumdioxid sowie Mischungen aus diesen Trägern verwendet.

Die Katalysatoren können nach bekannten Verfahren, z.B. durch Fällung, Auffällung, Imprägnierung, hergestellt werden.

Heterogenkatalysatoren für die Aminierung des eingesetzten Bio-Ethanols enthalten in ihrer katalytisch aktiven Masse vor der Behandlung mit Wasserstoff
20 bis 65 Gew.-%, besonders bevorzugt 22 bis 40 Gew.-%, $Al_2O_3$ und/oder $ZrO_2$
1 bis 30 Gew.-%, besonders bevorzugt 2 bis 25 Gew.-%, sauerstoffhaltige Verbindungen des Kupfers, berechnet als CuO, und
21 bis 70 Gew.-%, bevorzugt 21 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist.

In einer weiteren Variante enthalten diese besonders bevorzugten Katalysatoren zusätzlich in ihrer katalytisch

aktiven Masse vor der Behandlung mit Wasserstoff
15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind insgesamt in Mengen von bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Bevorzugte Heterogenkatalysatoren im erfindungsgemäßen Verfahren sind ausgewählt aus den

in EP-A-382 049 (BASF AG) offenbarten Katalysatoren, deren katalytisch aktive Masse vor der Behandlung mit Wasserstoff
20 bis 85 Gew.-%, bevorzugt 70 bis 80 Gew.-%, $ZrO_2$,
1 bis 30 Gew.-%, bevorzugt 1 bis 10 Gew.-%, CuO,
und jeweils 1 bis 40 Gew.-%, bevorzugt 5 bis 20 Gew.-%, CoO und NiO enthält, beispielsweise den in loc. cit. auf Seite 6 beschriebenen Katalysatoren mit der Zusammensetzung 76 Gew.-% Zr, berechnet als $ZrO_2$, 4 Gew.-% Cu, berechnet als CuO,
10 Gew.-% Co, berechnet als CoO, und 10 Gew.-% Ni, berechnet als NiO,
und insbesondere den in EP-A-963 975 (BASF AG) offenbarten Katalysatoren, deren katalytisch aktive Masse vor der Behandlung mit Wasserstoff
22 bis 40 Gew.-% $ZrO_2$,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als $Al_2O_3$ bzw. $MnO_2$,
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet als $ZrO_2$, 28 Gew.-% Ni, berechnet als NiO, 11 Gew.-% Cu, berechnet als CuO und 28 Gew.-% Co, berechnet als CoO.

Die hergestellten Katalysatoren können als solche gelagert werden. Vor ihrem Einsatz als Katalysatoren im erfindungsgemäßen Verfahren werden sie durch Behandlung mit Wasserstoff vorreduziert (= Aktivierung des Katalysators). Sie können jedoch auch ohne Vorreduktion eingesetzt werden, wobei Sie dann unter den Bedingungen des erfindungsgemäßen Verfahrens durch den im Reaktor vorhandenen Wasserstoff reduziert (= aktiviert) werden.

Zur Aktivierung wird der Katalysator bei einer Temperatur im Bereich von 100 bis 500°C, besonders 150 bis 400°C, ganz besonders 180 bis 300°C über einen Zeitraum von mindestens 25 Min., besonders mindestens 60 Min., einer Wasserstoff-haltigen Atmosphäre oder einer Wasserstoff-Atmosphäre ausgesetzt. Der Zeitraum der Aktivierung des Katalysators kann bis zu 1 h, besonders bis zu 12 h, insbesondere bis zu 24 h, ganz besonders bis zu 100 h, betragen und z.B. im Bereich von 24 bis 72 h liegen.

Bei dieser Aktivierung wird zumindest ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Der im erfindungsgemäßen Verfahren eingesetzte, mit Wasserstoff aktivierte Katalysator weist eine CO-Aufnahmekapazität von > 110 μmol CO / g Katalysator auf.
Ganz besonders weist er eine CO-Aufnahmekapazität vor > 120 μmol CO / g Katalysator, besonders bevorzugt > 130 μmol CO / g Katalysator, auf.
Die CO-Aufnahmekapazität kann z.B. bis zu 1000 μmol CO / g Katalysator, bevorzugt bis zu 500 μmol CO / g Katalysator, betragen.

Um CO-Aufnahmekapazität des Katalysators zu, bestimmen, wird er nach seiner Aktivierung mit Kohlenmonoxid (CO) behandelt und die CO-Chemisorption (= CO-Aufnahmekapazität) wie folgt gemessen.

Messung der CO-Aufnahmekapazität:

Eine Katalysatorprobe (ca. 0,3 g) wird in einem Probenröhrchen mit einer Rate von 5°C/Min auf 220°C erhitzt, während Wasserstoff mit einer Flussrate von 50 ml/Min. für 60 Min. über die Probe geleitet wird. Anschließend wird die Probe bei 220°C mit einem Heliumstrom (Flussrate: 50 ml/Min.) für 60 Min. gespült. Nach dieser Vorbehandlung wird die Chemisorption-Analyse bei 30°C unter Verwendung von 20 Vol.% CO in Helium nach der Methode gemäß DIN 66136-3 (CO-Puls-Chemisorption) durchgeführt. Der angenommene Stöchiometrie-Faktor für CO ist 1. Die adsorbierte CO-Menge wird bestimmt gemäß DIN 66136-3.

Das erfindungsgemäße Verfahren eignet sich beispielsweise zur Herstellung von Ethylaminen der Formel I

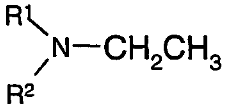

(I),

in der

$R^1$, $R^2$ Wasserstoff (H), Alkyl, wie $C_{1-200}$-Alkyl, Cycloalkyl, wie $C_{3-12}$-Cycloalkyl, Hydroxyalkyl, wie $C_{1-20}$-Hydroxyalkyl, Aminoalkyl, wie $C_{1-20}$-Aminoalkyl, Hydroxyalkylaminoalkyl, wie $C_{2-20}$-Hydroxyalkylaminoalkyl, Alkoxyalkyl, wie $C_{2-30}$-Alkoxyalkyl, Dialkylaminoalkyl, wie $C_{3-30}$-Dialkylaminoalkyl, Alkylaminoalkyl, wie $C_{2-30}$-Alkylaminoalkyl, Aryl, Heteroaryl, Aralkyl, wie $C_{7-20}$-Aralkyl, und Alkylaryl, wie $C_{7-20}$-Alkylaryl, oder gemeinsam $-(CH_2)_j-X-(CH_2)_k-$,

X $CH_2$, $CHR^3$, Sauerstoff (O), Schwefel (S) oder $NR^3$,

$R^3$ Wasserstoff (H), Alkyl, wie $C_{1-4}$-Alkyl, Alkylphenyl, wie $C_{7-40}$-Alkylphenyl, und

j, k eine ganze Zahl von 1 bis 4

bedeuten.

Das erfindungsgemäße Verfahren findet daher bevorzugt zur Herstellung eines Ethylamins I Anwendung, indem man das Bio-Ethanol mit einer Stickstoffverbindung der Formel II

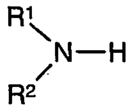

(II),

wobei $R^1$ und $R^2$ die oben genannten Bedeutungen haben, umsetzt.

Zur Herstellung des Ethylamins I wird demnach rein formal ein Wasserstoffatom der Stickstoffverbindung II durch den Rest $CH_3CH_2$- unter Freisetzung von einem Moläquivalent Wasser ersetzt.

Die Substituenten $R^1$ bis $R^3$, die Variable X und die Indizes j, k in den Verbindungen I und II haben unabhängig voneinander folgende Bedeutungen:

$R^1$, $R^2$:

- Wasserstoff (H),
- Alkyl, wie $C_{1-200}$-Alkyl, bevorzugt $C_{1-20}$-Alkyl, besonders bevorzugt $C_{1-14}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, isoHexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, insbesondere $C_{1-4}$-Alkyl,
- Cycloalkyl, wie $C_{3-12}$-Cycloalkyl, bevorzugt $C_{3-8}$-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,
- Hydroxyalkyl, wie $C_{1-20}$-Hydroxyalkyl, bevorzugt $C_{1-8}$-Hydroxyalkyl, besonders bevorzugt $C_{1-4}$-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,
- Aminoalkyl, wie $C_{1-20}$-Aminoalkyl, bevorzugt $C_{1-8}$-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,

- Hydroxyalkylaminoalkyl, wie $C_{2-20}$-Hydroxyalkylaminoalkyl, bevorzugt $C_{3-8}$-Hydroxyalkylaminoalkyl, wie (2-Hydroxyethylamino)methyl, 2-(2-Hydroxyethylamino)ethyl und 3-(2-Hydroxyethylamino)propyl,

- Alkoxyalkyl, wie $C_{2-30}$-Alkoxyalkyl, bevorzugt $C_{2-20}$-Alkoxyalkyl, besonders bevorzugt $C_{2-8}$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt $C_{2-4}$-Alkoxyalkyl,

- Dialkylaminoalkyl, wie $C_{3-30}$-Dialkylaminoalkyl, bevorzugt $C_{3-20}$-Dialkylaminoalkyl, besonders bevorzugt $C_{3-10}$-N,N-Dialkylaminoalkyl, wie (N,N-Dimethylamino)-methyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl, 2-(N,N-Di-iso-propylamino)ethyl, $(R^3)_2N-(CH_2)_q$ (q =1 bis 6), ganz besonders 3-(N,N-Dimethylamino)propyl

- Alkylaminoalkyl, wie $C_{2-30}$-Alkylaminoalkyl, bevorzugt $C_{2-20}$-Alkylaminoalkyl, besonders bevorzugt $C_{2-8}$-Alkylaminoalkyl, wie Methylaminomethyl, 2-(Methylamino)ethyl, Ethylaminomethyl, 2-(Ethylamino)ethyl und 2-(iso-Propylamino)-ethyl, $(R^3)HN-(CH_2)_q$ (q = 1 bis 6),

- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

- Aralkyl, wie $C_{7-20}$-Aralkyl, bevorzugt $C_{7-12}$-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

- Alkylaryl, wie $C_{7-20}$-Alkylaryl, bevorzugt $C_{7-12}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

- oder beide Reste bedeuten gemeinsam eine $-(CH_2)_j-X-(CH_2)_k-$ Gruppe, wie - $(CH_2)_3-$, $-(CH_2)_4-$, $-(CH_2)_5-$, $-(CH_2)_6-$, $-(CH_2)_7-$, $-(CH_2)-O-(CH_2)_2-$, $-(CH_2)-NR^3-(CH_2)_2-$, $-(CH_2)-CHR^3-(CH_2)_2-$, $-(CH_2)_2-O-(CH_2)_2-$, $-(CH_2)_2-NR^3-(CH_2)_2-$, $-(CH_2)_2-CHR^3-(CH_2)_2-$, $-CH_2-O-(CH_2)_3-$, $-CH_2-NR^3-(CH_2)_3-$,

$R^3$:

- Wasserstoff (H),

- Alkyl, besonders $C_{1-4}$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

- Alkylphenyl, besonders $C_{7-40}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,

X:

- $CH_2$, $CHR^3$, Sauerstoff (O), Schwefel (S) oder $NR^3$, bevorzugt $CH_2$, NH und O,

j:

- eine ganze Zahl von 1 bis 4 (1, 2, 3 oder 4), bevorzugt 1 und 2, und

k:

- eine ganze Zahl von 1 bis 4 (1, 2, 3 oder 4), bevorzugt 1 und 2.

Als Aminierungsmittel bei der hydrierenden Aminierung des Bio-Ethanols in Gegenwart von Wasserstoff können

sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe zunächst in die primäre Aminogruppen (-$NH_2$) umgewandelt. Das so gebildete primäre Ethylamin kann mit weiterem Bio-Ethanol zu dem entsprechenden sekundären Amin (Diethylamin) und dieses wiederum mit weiterem Alkohol zu dem entsprechenden tertiären Amin (Triethylamin) reagieren. Je nach Zusammensetzung des Reaktionsansatzes oder des Eduktstroms (bei kontinuierlicher Fahrweise) und je nach den angewandten Reaktionsbedingungen - Druck, Temperatur, Katalysator, Reaktionszeit (Katalysatorbelastung) - lassen sich auf diese Weise je nach Wunsch bevorzugt primäre, sekundäre oder tertiäre Ethylamine darstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet.

Beispielsweise werden die folgenden Mono- und Dialkylamine als Aminierungsmittel verwendet: Methylamin, Dimethylamin, Ethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel Monoethylamin (aus Ethanol und Ammoniak), Diethylamin (aus Ethanol und Monoethylamin), Triethylamin (aus Ethanol und Diethylamin), Mono-/Di-/Triethylamin-Gemisch (aus Ethanol und Ammoniak) und Dimethylethylamin (aus Ethanol und Dimethylamin).

Das Aminierungsmittel kann bezüglich der zu aminierenden alkoholischen Hydroxylgruppe in stöchiometrischen, unter- oder überstöchiometrischen Mengen eingesetzt werden.

Bevorzugt wird im Falle der Aminierung mit primären oder sekundären Aminen das Amin in ca. stöchiometrischer Menge oder geringfügig überstöchiometrischer Menge pro Mol alkoholischer Hydroxylgruppe eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe eingesetzt.

Höhere Überschüsse sowohl an Ammoniak als auch an primären oder sekundären Aminen sind möglich.

Das erfindungsgemäße Verfahren lässt sich diskontinuierlich oder bevorzugt kontinuierlich wie folgt durchführen, wobei der Katalysator bevorzugt als Festbett im Reaktor angeordnet ist.
Die Ausführungsform als Wirbelbettreaktion mit in auf- und abwirbelnder Bewegung befindlichem Katalysatormaterial ist jedoch ebenfalls möglich.

Die Aminierung kann in der Flüssigphase oder in der Gasphase durchgeführt werden. Bevorzugt ist das Festbettverfahren in der Gasphase.

Beim Arbeiten in der Flüssigphase leitet man den die Edukte (Alkohol plus Ammoniak oder Amin) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 300°C, bevorzugt 120 bis 270°C, besonders bevorzugt 130 bis 250°C, insbesondere 170 bis 230°C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol plus Ammoniak oder Amin) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 7 MPa, in Gegenwart von Wasserstoff über den Katalysator geleitet. Die Temperaturen für die Aminierung betragen im allgemeinen 80 bis 300°C, bevorzugt 120 bis 270°C, besonders bevorzugt 160 bis 250°C. Es ist dabei sowohl eine Anströmung des Katalysatorfestbetts von oben als auch von unten möglich. Den erforderlichen Gasstrom erhält man bevorzugt durch eine Kreisgasfahrweise.
Die Katalysatorbelastung liegt im allgemeinen im Bereich von 0,01 bis 2, bevorzugt 0,05 bis 0,5, kg Alkohol pro Liter Katalysator (Schüttvolumen) und Stunde.

Der Wasserstoff wird der Reaktion im allgemeinen in einer Menge von 5 bis 400 l, bevorzugt in einer Menge von 50 bis 200 l pro Mol Alkoholkomponente zugeführt, wobei die Literangaben jeweils auf Normalbedingungen umgerechnet wurden (S.T.P.).

Sowohl beim Arbeiten in der Flüssigphase als auch beim Arbeiten in der Gasphase ist die Anwendung höherer Temperaturen und höherer Gesamtdrücke möglich. Der Druck im Reaktionsgefäß, welcher sich aus der Summe der Partialdrücke des Aminierungsmittels, des Alkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z.B. destillativ.

Aus dem Reaktionsaustrag werden, nachdem dieser zweckmäßigerweise entspannt worden ist, das überschüssige Aminierungsmittel und der Wasserstoff entfernt und die erhaltenen Aminierungsprodukte (Ethylamine) durch Destillation bzw. Rektifikation, Flüssigextraktion oder Kristallisation gereinigt. Das überschüssige Aminierungsmittel und der Wasserstoff werden vorteilhaft wieder in die Reaktionszone zurückgeführt. Das gleiche gilt für eventuell nicht vollständig umgesetzten Bio-Alkohol.

Die unter Anwendung des erfindungsgemäßen Verfahrens hergestellten Amine eignen sich u. a. als Zwischenprodukte bei der Herstellung von Kraftstoffadditiven (US-A-3,275,554; DE-A-21 25 039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

Alle ppm-Angaben in diesem Dokument beziehen sich auf das Gewicht.

Beispiele

Katalysator A:

Zusammensetzung: 4 Gew.-% CuO, 10 Gew.-% CoO, 10 Gew.-% NiO, Rest: $Al_2O_3$. CO-Aufnahmekapazität: 36 μmol CO / g Katalysator.

Katalysator B (erfindungsgemäß):

Zusammensetzung: 13 Gew.-% CuO, 28 Gew.-% CoO, 28 Gew.-% NiO, Rest: $ZrO_2$. CO-Aufnahmekapazität: 135 μmol CO / g Katalysator.

Die CO-Aufnahmekapazität wurde bestimmt wie oben auf Seite 6, Zeilen 27 bis 39 beschrieben.

Von den Katalysatoren A und B wurden jeweils 2,0 kg bzw. 2,8 kg in einen 5 Liter-Reaktor eingebaut und im Wasserstoffstrom wie folgt aktiviert.

Katalysator B: In 12 h mit 200 Nl/h $H_2$ auf 280°C aufheizen und 24 h bei 280°C drucklos reduzieren. Danach Einstellung auf Syntheseparameter.

Katalysator A: Mit 100 Nl/h $H_2$ innerhalb von 24 h auf 280°C aufheizen und 48 h bei 280°C mit 100 Nl/h $H_2$ drucklos reduzieren. Danach Einstellung auf Syntheseparameter. (Nl = Normliter = auf Normalbedingungen umgerechnetes Volumen).

Es wurde bei einem Druck von 66 bar mit Ammoniak vorgefahren und danach Bio-Ethanol in den Gasstrom kontinuierlich dosiert, so dass ein konstantes Mol-Verhältnis Ammoniak : Bio-Ethanol von 1,4 : 1 eingestellt war. Die Reaktortemperatur wurde derart eingestellt, dass der kondensierte Austrag neben Mono-, Di- und Triethylamin Rest-Bio-Ethanol von nicht mehr als etwa 3 Gew.-% enthielt.

Am Katalysator A musste dafür eine Anfangstemperatur von 195°C eingestellt werden. Während im weiteren Verlauf 135 kg Bio-Ethanol / kg Katalysator A kontinuierlich über

den Katalysator gegeben wurden, musste die Temperatur schrittweise auf 227°C nachgeregelt werden, um im Austrag den Ethanolgehalt unterhalb von 3 Gew.-% zu halten.

Am Katalysator B reichte unter ansonsten identischen Bedingungen die Einstellung einer Anfangstemperatur von 175°C im Katalysatorbett aus, um im Austrag weniger als 3 Gew.-% Ethanol zu haben. Auch nach 135 kg Bio-Ethanol / kg Katalysator B zeigte der Katalysator bei 175°C keinen Aktivitätsabfall.

Beispiele für kommerzielle Bio-Ethanol-Qualitäten

Verschiedene kommerzielle Bio-Ethanol-Qualitäten wurden untersucht auf ihren Schwefelgehalt.

| | Bio-EtOH 1 | Bio-EtOH 2 | Bio-EtOH 3 | Bio-EtOH 4 | Bio-EtOH 5 | Bio-EtOH 6 | Bio-EtOH 7 |
|---|---|---|---|---|---|---|---|
| Ges.-S (Gew.-ppm) | 0,6 | 1 | 0,6 | 8 | 2 | 49 | 2 |
| Sulfat-S (Gew.-ppm) | 0,33 | 0,43 | 0,2 | n.b. | 0,9 | 6 | 2 |

Ges.-S = Gesamt-Schwefel (= Gehalt an Schwefel und/oder schwefelhaltigen Verbindungen, berechnet S), Gehalte > 2 ppm wurden coulometrisch bestimmt nach DIN 51400 Teil 7,
Gesamt-Schwefel-Gehalte ≤ 2 ppm wurden bestimmt nach Wickbold (DIN EN 41).
Sulfat-S = Sulfat-Schwefel, ionenchromatographisch bestimmt analog EN ISO 10304-2.
n.b. = nicht bestimmt.

**Patentansprüche**

1. Verfahren zur Herstellung eines Ethylamins durch Umsetzung von Ethanol mit Ammoniak, einem primären Amin oder einem sekundären Amin in Gegenwart von Wasserstoff und eines Heterogen-Hydrier-/Dehydrier-Katalysators, **dadurch gekennzeichnet, dass** man ein biochemisch oder biologisch hergestelltes Ethanol (Bio-Ethanol) einsetzt, der Katalysator in seiner katalytisch aktiven Masse vor der Aktivierung mit Wasserstoff 20 bis 65 Gew.-% Zirkoniumdioxid ($ZrO_2$) und/oder Aluminiumoxid ($Al_2O_3$) als Trägermaterial, 1 bis 30 Gew.-% sauerstoffhaltige Verbindungen des Kupfers, berechnet als CuO, und 21 bis 70 Gew.-% sauerstoffhaltige Verbindungen des Nickels, berechnet als NiO, enthält und nach Aktivierung mit Wasserstoff eine CO-Aufnahmekapazität von > 110 µmol CO / g Katalysator aufweist, wobei die Aktivierung des Katalysators mit Wasserstoff bei einer Temperatur im Bereich von 100 bis 500°C über einen Zeitraum von mindestens 25 Min. erfolgt.

2. Verfahren nach dem vorhergehenden Anspruch zur Herstellung von Mono-, Di- und/oder Triethylamin durch Umsetzung des Ethanols mit Ammoniak.

3. Verfahren nach einem der beiden vorhergehenden Ansprüche, **dadurch gekennzeichnet, dass** man ein fermentativ hergestelltes Ethanol einsetzt.

4. Verfahren nach einem der vorhergehenden Ansprüche, **dadurch gekennzeichnet, dass** der Katalysator nach Aktivierung mit Wasserstoff eine CO-Aufnahmekapazität von > 120 µmol CO / g Katalysator aufweist.

5. Verfahren nach einem der Ansprüche 1 bis 3, **dadurch gekennzeichnet, dass** der Katalysator nach Aktivierung mit Wasserstoff eine CO-Aufnahmekapazität von > 130 µmol CO / g Katalysator aufweist.

6. Verfahren nach einem der vorhergehenden Ansprüche, **dadurch gekennzeichnet, dass** der Katalysator Cu, Co und Ni enthält.

7. Verfahren nach einem der vorhergehenden Ansprüche, **dadurch gekennzeichnet**, das der Katalysator in seiner katalytisch aktiven Masse vor der Aktivierung mit Wasserstoff 20 bis 65 Gew.-% Zirkoniumdioxid ($ZrO_2$) und/oder Aluminiumoxid ($Al_2O_3$) als Trägermaterial, 1 bis 30 Gew.-% sauerstoffhaltige Verbindungen des Kupfers, berechnet als CuO, 21 bis 50 Gew.-% sauerstoffhaltige Verbindungen des Nickels, berechnet als NiO, und 15 bis 50 Gew.-% sauerstoffhaltige Verbindungen des Kobalts, berechnet als CoO, enthält.

8. Verfahren nach einem der vorhergehenden Ansprüche, **dadurch gekennzeichnet, dass** man ein Bio-Ethanol eingesetzt, das einen Gehalt an Schwefel und/oder schwefelhaltigen Verbindungen von ≥ 0,5 Gew.-ppm (berechnet S) aufweist.

9. Verfahren nach einem der vorhergehenden Ansprüche, **dadurch gekennzeichnet, dass** man die Umsetzung bei einer Temperatur im Bereich von 80 bis 300°C durchführt.

10. Verfahren nach einem der vorhergehenden Ansprüche, **dadurch gekennzeichnet, dass** man die Umsetzung in der Flüssigphase bei Drücken im Bereich von 5 bis 30 MPa oder in der Gasphase bei Drücken im Bereich von 0,1

bis 40 MPa durchführt.

**11.** Verfahren nach einem der vorhergehenden Ansprüche, **dadurch gekennzeichnet, dass** die Aktivierung des Katalysators mit Wasserstoff bei einer Temperatur im Bereich von 150 bis 400°C über einen Zeitraum von mindestens 60 Min. erfolgt.

## Claims

**1.** A process for preparing an ethylamine by reacting ethanol with ammonia, a primary amine or a secondary amine in the presence of hydrogen and a heterogeneous hydrogenation/dehydrogenation catalyst, wherein a biochemically or biologically prepared ethanol (bioethanol) is used and the catalytically active composition of the catalyst prior to activation with hydrogen comprises from 20 to 65% by weight of zirconium dioxide ($ZrO_2$) and/or aluminum oxide ($Al_2O_3$) as support material, from 1 to 30% by weight of oxygen-comprising compounds of copper, calculated as CuO, and from 21 to 70% by weight of oxygen-comprising compounds of nickel, calculated as NiO, and after activation with hydrogen has a CO uptake capacity of > 110 μmol of CO/g of catalyst, with the activation of the catalyst with hydrogen being carried out at a temperature in the range from 100 to 500°C over a period of at least 25 minutes.

**2.** The process according to the preceding claim for preparing monoethylamine, diethylamine and/or triethylamine by reacting ethanol with ammonia.

**3.** The process according to either of the two preceding claims, wherein an ethanol prepared by fermentation is used.

**4.** The process according to any of the preceding claims, wherein the catalyst after activation with hydrogen has a CO uptake capacity of > 120 μmol of CO/g of catalyst.

**5.** The process according to any of claims 1 to 3, wherein the catalyst after activation with hydrogen has a CO uptake capacity of > 130 μmol of CO/g of catalyst.

**6.** The process according to any of the preceding claims, wherein the catalyst comprises Cu, Co and Ni.

**7.** The process according to any of the preceding claims, wherein the catalytically active composition of the catalyst prior to activation with hydrogen comprises from 20 to 65% by weight of zirconium dioxide ($ZrO_2$) and/or aluminum oxide ($Al_2O_3$) as support material, from 1 to 30% by weight of oxygen-comprising compounds of copper, calculated as CuO, from 21 to 50% by weight of oxygen-comprising compounds of nickel, calculated as NiO, and from 15 to 50% by weight of oxygen-comprising compounds of cobalt, calculated as CoO.

**8.** The process according to any of the preceding claims, wherein a bioethanol which has a content of sulfur and/or sulfur-comprising compounds of ≥ 0.5 ppm by weight (calculated as S) is used.

**9.** The process according to any of the preceding claims, wherein the reaction is carried out at a temperature in the range from 80 to 300°C.

**10.** The process according to any of the preceding claims, wherein the reaction is carried out in the liquid phase at pressures in the range from 5 to 30 MPa or in the gas phase at pressures in the range from 0.1 to 40 MPa.

**11.** The process according to any of the preceding claims, wherein the activation of the catalyst with hydrogen is carried out at a temperature in the range from 150 to 400°C over a period of at least 60 minutes.

## Revendications

**1.** Procédé de fabrication d'une éthylamine en faisant réagir de l'éthanol avec de l'ammoniac, une amine primaire ou une amine secondaire, en présence d'hydrogène et d'un catalyseur hétérogène d'hydrogénation/déshydrogénation, **caractérisé en ce que** l'on utilise un éthanol (bioéthanol) fabriqué par voie biochimique ou biologique, dans lequel le catalyseur contient dans sa masse active au plan catalytique, avant activation avec de l'hydrogène, 20 à 65 % en poids de dioxyde de zirconium ($ZrO_2$) et/ou d'oxyde d'aluminium ($Al_2O_3$) comme matériau de support, 1 à 30 %

en poids de composés de cuivre contenant de l'oxygène, calculés comme CuO, et 21 à 70 % en poids de composés de nickel contenant de l'oxygène, calculés comme NiO, et présente, après activation avec de l'hydrogène, une capacité d'absorption de CO > 110 µmoles de CO/g de catalyseur, l'activation du catalyseur avec l'hydrogène s'effectuant à une température dans la plage de 100 à 500 °C sur une période de temps d'au moins 25 min.

**2.** Procédé selon la revendication précédente, pour la fabrication de mono-, di- et/ou triéthylamine, en faisant réagir de l'éthanol avec de l'ammoniac.

**3.** Procédé selon l'une quelconque des deux revendications précédentes, **caractérisé en ce que** l'on utilise un éthanol fabriqué par fermentation.

**4.** Procédé selon l'une quelconque des revendications précédentes, **caractérisé en ce que** le catalyseur présente, après activation avec de l'hydrogène, une capacité d'absorption de CO > 120 µmoles de CO/g de catalyseur.

**5.** Procédé selon l'une quelconque des revendications 1 à 3, **caractérisé en ce que** le catalyseur présente, après activation avec de l'hydrogène, une capacité d'absorption de CO > 130 µmoles de CO/g de catalyseur.

**6.** Procédé selon l'une quelconque des revendications précédentes, **caractérisé en ce que** le catalyseur contient les éléments Cu, Co et Ni.

**7.** Procédé selon l'une quelconque des revendications précédentes, **caractérisé en ce que** le catalyseur contient dans sa masse active au plan catalytique, avant activation avec de l'hydrogène, 20 à 65 % en poids de dioxyde de zirconium ($ZrO_2$) et/ou d'oxyde d'aluminium ($Al_2O_3$) comme matériau de support, 1 à 30 % en poids de composés de cuivre contenant de l'oxygène, calculés comme CuO, 21 à 50 % en poids de composés de nickel contenant de l'oxygène, calculés comme NiO, et 15 à 50 % en poids de composés de cobalt contenant de l'oxygène, calculés comme CoO.

**8.** Procédé selon l'une quelconque des revendications précédentes, **caractérisé en ce que** l'on utilise un bioéthanol qui présente une teneur en soufre et/ou en composés contenant du soufre ≥ 0,5 ppm en poids (calculés comme S).

**9.** Procédé selon l'une quelconque des revendications précédentes, **caractérisé en ce que** l'on effectue la réaction à une température dans la plage de 80 à 300 °C.

**10.** Procédé selon l'une quelconque des revendications précédentes, **caractérisé en ce que** l'on effectue la réaction en phase liquide à des pressions dans la plage de 5 à 30 MPa ou en phase gazeuse à des pressions dans la plage de 0,1 à 40 MPa.

**11.** Procédé selon l'une quelconque des revendications précédentes, **caractérisé en ce que** l'activation du catalyseur avec de l'hydrogène s'effectue à une température dans la plage de 150 à 400 °C sur une période de temps d'au moins 60 min.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 04014591 W
- EP 04014588 W
- DE 102005010328
- US 4760190 A
- EP 382049 A
- EP 963975 A
- US 3275554 A
- DE 2125039 A
- DE 3611230 A


### In der Beschreibung aufgeführte Nicht-Patentliteratur


- Ullmann’s Encyclopedia of Industrial Chemistry. 2000
- **K. WEISSERMEL ; H.-J. ARPE.** Industrial Organic Chemistry. Wiley-VCH, 2003, 194